# EUROPÄISCHE PATENTANMELDUNG

(11) **EP 1 625 834 A2**
(43) Veröffentlichungstag der Anmeldung: **15.02.2006**
(21) Anmeldenummer: 05405442.4
(22) Anmeldetag: 18.07.2005
(51) Int. Cl.: A61F 2/16

(54) **Vorrichtung zum Beladen einer Linsenfaltkartusche mit einer intraokularen Linse, sowie Linsenfaltkartusche und Set für die Implantation**

(30) Priorität: 12.08.2004 CH 133404
(71) Anmelder: Medicel AG, 9443 Widnau (CH)
(72) Erfinder: Hohl, Emil, 9435 Heerbrugg (CH)
(74) Vertreter: Riederer, Conrad A.

(57) **Zusammenfassung**

Die Vorrichtung (10) zum Beladen einer Linsenfaltkartusche (11) mit einer intraokularen Linse (13) weist einen Support (41) auf, welcher eine Aussparung (43) zur Aufnahme der noch leeren Linsenfaltkartusche (11) aufweist. Der Führung und Halterung der Linsenfaltkartusche (11) dienen eine zylindrische Oeffnung (45), welche den hinteren Teil (20) der Injektordüse (19) umfasst und eine Nut (47), welche den Flügel (27) festhält. Durch die zylindrische Oeffnung (45) erstreckt sich ein Haken (45) nach aussen. Wird somit die Linsenfaltkartusche (11) in die Vorrichtung geschoben, erstreckt sich der Haken (49) durch die Injektordüse (19) hindurch bis in den vorderen Bereich der Halbschale (21). Die Linse (13) wird nun so in die Linsenfaltkartusche (11) gelegt, dass die vordere Haptik (39) vom Haken (49) erfasst werden kann und die hintere Haptik (37) in der Kerbe (35) liegt. In dieser Form liegt dem Operateur die Vorrichtung (10) mit Linsenfaltkartusche (11) und Linse (13) steril verpackt vor. Zum Gebrauch entnimmt er die Vorrichtung (10) und verschwenkt den Flügel (29), bis er in der Schnappeinrichtung (33) einschnappt. Die Linse (13) ist nun gefaltet. Beim Herausziehen der Linsenfaltkartusche (11) wird die vordere Haptik (39) durch den Haken (49) in die Injektordüse (19) geleitet.

## Beschreibung

Die vorliegende Erfindung betrifft eine Vorrichtung zum Beladen einer Linsenfaltkartusche mit einer intraokularen Linse.

Bei Kataraktoperationen werden heutzutage standardmässig künstliche Linsen, sogenannte intraokulare Linsen, in den Kapselsack des Auges eingesetzt. Zum Einsetzen einer intraokularen Linse sind in den vergangenen Jahren sogenannte Kartuschen entwickelt worden, in welche die Linse geladen und sodann mittels eines Injektors aus der Kartusche ausgestossen werden kann. Beispiele für solche Kartuschen und Injektoren sind aus den US 6,283,975 und WO 03/045285 bekannt.

Die üblichen Kartuschen, wie sie beispielsweise in der EP 1023 880 beschrieben werden, weisen einen Ladekanal auf, der aus zwei Halbschalen besteht, welche mittels eines Filmscharniers verbunden sind. In diese Halbschalen kann die intraokulare Linse eingelegt werden. An den Halbschalen sind Flügel angeordnet, welche das Schliessen der Kartusche und damit das Falten der Linse erleichtern. Vom am Ladekanal befindet sich eine Injektordüse, durch welche die gefaltete Linse nach dem Einlegen der Kartusche in einen Injektor ausgestossen werden kann.

Das richtige Einlegen der Linse in die Kartusche erfordert vom Anwender viel Geschick. In der WO 03/045285 wird deshalb ein Set vorgeschlagen, bei dem eine intraokulare Linse in spannungsfreiem Zustand bereits in einer Kartusche enthalten ist, welche zur Faltung der Linse nur noch geschlossen werden muss. Diese Kartusche wird durch einen Halter getragen und ist bis zur Verwendung in einer Packung steril verpackt, und zwar im Fall einer hydrophilen Linse in einer Flüssigkeit, welche die Linse vor dem Austrocknen schützt. Bei der Operation wird die Kartusche samt der darin gelagerten Linse der Packung entnommen, gefaltet und in den Injektor eingesetzt. Nach dem Einfüllen einer viskoelastischen Flüssigkeit in den Injektionskanal der Kartusche ist das System zur Injektion der intraokularen Linse in den Kapselsack des zu behandelnden Auges bereit.

Es hat sich gezeigt, dass auch die Handhabung der beschriebenen Kartuschen durch den Anwender recht viel Geschick erfordert, weil die Kartuschen klein dimensioniert sind. Besonders heikel erweisen sich dreiteilige intraokulare Linsen mit filamentären Haptiken (filamentary haptics).

Es ist daher Aufgabe der vorliegenden Erfindung, Wege und Mittel aufzuzeigen, um die Handhabung der Linsenfaltkartuschen zu erleichtern und insbesondere eine Beschädigung der Linse, insbesondere deren Haptiken, zu vermeiden.

Zur Lösung dieser Aufgabe sieht die Erfindung eine Ladevorrichtung zum Beladen einer Linsenfaltkartusche mit einer intraokularen Linse vor. Diese Ladevorrichtung ist dadurch gekennzeichnet, dass sie eine Auflage zur Aufnahme der Linsenfaltkartusche besitzt. In diese Vorrichtung kann bereits der Hersteller der Linse die Linse in ungefaltetem Zustand in der optimalen Lage platzieren. Der Anwender muss dann nur noch einen Flügel der Linsenfaltkartusche verschwenken, um die Linse zu falten. Dies ist leicht zu bewerkstelligen, weil sich die Linsenfaltkartusche in einer Vorrichtung befindet, die grösser als die Linsenfaltkartusche ist, was die Handhabung wesentlich erleichtert.

Die Vorrichtung besitzt vorteilhaft einen Support aus Kunststoff, in welchem die Auflage für die Linsenfaltkartusche durch eine Aussparung gebildet ist. Zweckmässigerweise weist der Support Führungsmittel zum Einführen der Linsenkartusche auf. Die Führungsmittel können eine Führung, z.B. eine Oeffnung, aufweisen, welche die Injektordüse führen. Eine zweite Führung, z.B. eine Nut, kann dazu dienen, die äusseren Enden eines der Flügel der Linsenfaltkartusche zu führen.

Durch diese Führungen wird nach dem Einschieben der Kartusche in die Ladevorrichtung die Kartusche festgehalten, wobei aber ein Flügel verschwenkbar bleibt.

Von ganz besonderem Vorteil erweist sich ein Haken, welcher sich durch die Führung für die Injektordüse hindurch nach aussen erstreckt. Führt man die Linsenfaltkartusche in die Vorrichtung ein, so erstreckt sich der Haken durch die Injektordüse hindurch bis hinein in den vorderen Teil des Ladekanals. Beim Einlegen einer Linse mit filamentären Haptiken in den Ladekanal wird darauf geachtet, dass die vordere filamentäre Haptik in eine Lage gebracht wird, in der sie bei der Entnahme der Kartusche aus der Ladevorrichtung vom Haken erfasst wird. Wenn nun später der Anwender die Linsenfaltkartusche faltet und der Ladevorrichtung entnimmt, wird durch den stationären Haken die vordere Haptik in die Injektordüse der Linsenfaltkartusche geleitet, und gestreckt.

Die Ladevorrichtung kann ein Verriegelungselement aufweisen, um die Linsenfaltkartusche nach dem Einlegen der intraokularen Linse in teiloffener Stellung zu halten. Dadurch wird sichergestellt, dass die Linse nicht herausfällt. Andererseits wird aber keine Spannung auf die Linse ausgeübt.

Zweckmässigerweise besitzt das Verriegelungselement einen Mitnehmer für die hintere Haptik, um beim Entriegeln die hintere Haptik richtig für den Injektionsvorgang zu platzieren. Zu diesem Zweck kann das Verriegelungselement auf einer Achse des Supports verschwenkbar gelagert sein. Zum Verschwenken kann das Verriegelungselement als manuell betätigbarer Schwenkarm ausgebildet sein. Dabei ist vorteilhaft der Mitnehmer für die hintere Haptik an diesem Schwenkarm ausgebildet. Das Verriegelungselement kann einen Anschlag aufweisen, um die Linsenfaltkartusche in teiloffener Stellung zu halten, in welcher die Linse spannungsfrei gehalten wird.

Die Linsenfaltkartusche zur Verwendung mit einer Ladevorrichtung der vorangehend beschriebenen Art besitzt einen Ladekanal bestehend aus zwei Halbschalen, die mittels eines Scharniers verbunden sind, an den Halbschalen angeordnete Flügel und eine vorn am Ladekanal angeordnete Injektordüse und ist dadurch gekennzeichnet, dass hinten an einer der Halbschalen eine Kerbe zur Aufnahme der hinteren Haptik angeordnet ist. Es wäre aber auch möglich, eine funktionell ebenbürtige Gestaltung mit zwei Filmscharnieren vorzusehen, wie dies beispielsweise in der US 5947975 gezeigt wird.

Die Erfindung betrifft auch ein Set für die Implantation einer intraokularen Linse. Dieses Set umfasst eine Linsenfaltkartusche und ist dadurch gekennzeichnet, dass es weiter eine Ladevorrichtung zum Laden der Linsenfaltkartusche umfasst. Vorteilhaft umfasst das Set weiter einen Injektor. Zweckmässigerweise sind die Ladevorrichtung mit der Linsenfaltkartusche und die von der Linsenfaltkartusche sicher gehaltene, aber ungefaltete intraokulare Linse in einer sterilen Verpackung gelagert.

Ausführungsbeispiele der Erfindung werden nun unter Bezugnahme auf die Zeichnung beschrieben. Es zeigt:
- Figur 1:: ein erstes Ausführungsbeispiel einer Ladevorrichtung zusammen mit einer offenen Linsenfaltkartusche und einer noch ungefalteten intraokularen Linse,
- Figur 2:: eine Linsenfaltkartusche, welche speziell für die Verwendung zusammen mit einer Ladevorrichtung gemäss Figur 1 ausgebildet ist,
- Figur 3:: eine andere Ansicht der Linsenfaltvorrichtung von Fig. 1 bei der Entnahme der geschlossenen Linsenfaltkartusche mit der gefalteten Linse, und
- Figur 4:: ein weiteres Ausführungsbeispiel einer Ladevorrichtung, wobei diese mit einem Verriegelungselement versehen ist.

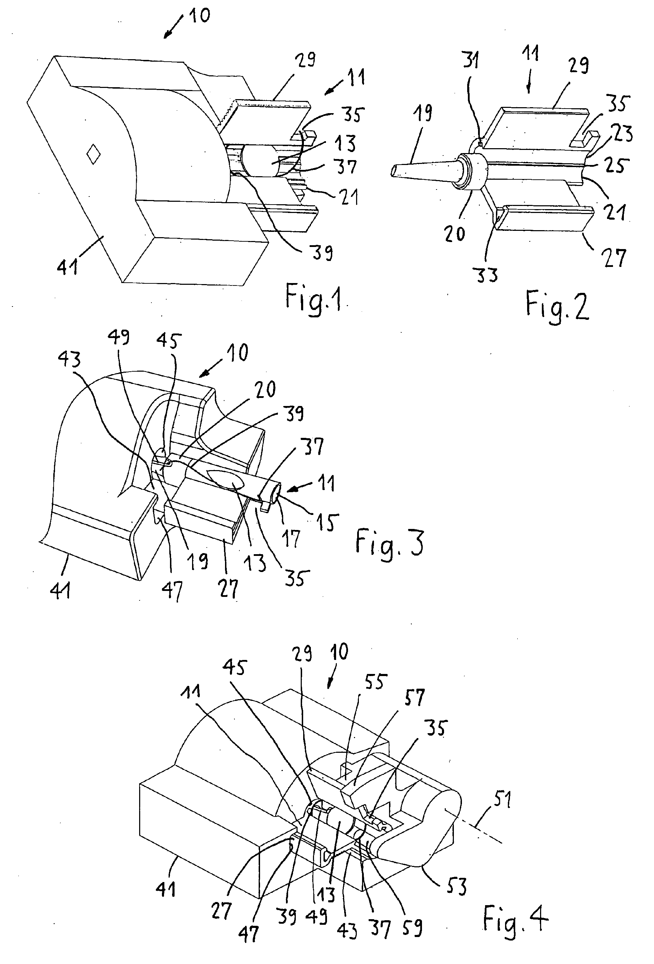

In den Figuren 1 und 3 wird eine Ladevorrichtung 10 zusammen mit einer Linsenfaltkartusche 11 gezeigt. Ersichtlich ist auch die intraokulare Linse 13, die sich in Figur 1 in ungefaltetem und in Figur 3 in gefaltetem Zustand befindet.

Die Linsenfaltkartusche 11, deren Gestaltung insbesondere aus Figur 2 ersichtlich ist, besteht vorzugsweise aus durchsichtigem Kunststoff und besitzt einen zylindrischen Ladekanal 15 (Fig. 3) mit einer Eintrittsöffnung 17 und einem fluchtend an den Ladekanal 15 sich anschliessende Injektordüse 19. Die Linsenfaltkartusche 11 ist faltbar. Der Ladekanal 15 besteht aus zwei zylindrischen Halbschalen 21,23, welche mittels eines Filmscharniers 25 zusammengehalten sind. An den Halbschalen 21,23 sind Flügel 27,29 ausgebildet. Auf der Innenseite jeder Halbschale 21,23 befindet sich eine Längsnut 31 (nur eine ist sichtbar). Beim Einlegen der Linse 13 in die Kartusche 11 werden die Linsenränder von den Längsnuten 31 erfasst. Durch die Anordnung der Längsnuten 31 ausserhalb der Mitte der Halbschalen 21,23 wird erreicht, dass sich die Linse 13 beim Falten der Kartusche immer gegen das Scharniergelenk 25 hin faltet. Eine Schnappvorrichtung 33 ist an einem Flügel 27 ausgebildet, damit nach dem Falten der Linse 13 die Kartusche in der Faltstellung verbleibt. Der bis jetzt beschriebene Aufbau entspricht dem bekannten Stand der Technik, wie er beispielsweise in der WO 03/045285 beschrieben wird. Im Gegensatz zum bekannten Stand der Technik weist jedoch die in Figur 2 gezeigte Kartusche 11 eine Kerbe 35 zur Aufnahme der hinteren Haptik 37 der Linse 13 auf. In den Figuren 1 und 3 ist die vordere Haptik mit dem Bezugszeichen 39 bezeichnet.

Die Ladevorrichtung 10 besteht im wesentlichen aus einem Support 41, der vorzugsweise aus Kunststoff besteht. Eine Aussparung 43 (Fig. 3) im Support 41 dient als Auflage zur Aufnahme der Linsenfaltkartusche 11. Führungsmittel 45,47 dienen dem Einführen der Linsenfaltkartusche 11. Die Oeffnung 45 führt den hinteren Teil 20 der Injektordüse 19 und die Nut 47 dient der Führung des äusseren Endes des Flügels 27 der Linsenfaltkartusche 11. Auf diese Weise wird die Linsenfaltkartusche 11 stabil in der Ladevorrichtung 10 festgehalten, wobei aber der Flügel 29 verschwenkbar bleibt (Fig. 1). Koaxial zur Oeffnung 45 erstreckt sich im Inneren des Supports 41 ein Haken 49 nach aussen (Fig. 3). Dieser Haken 49 hat die Aufgabe, bei der Entnahme der verschlossenen Linsenfaltkartusche 11 die vordere Haptik 39 richtig in der Injektordüse 19 zu platzieren. Dadurch wird später eine Beschädigung der Haptik 39 beim Ausstossen der Linse 13 durch die Injektordüse 19 vermieden. Wenn die Ladevorrichtung für einteilige Linsen, also für Linsen ohne filamentäre Haptiken verwendet wird, kann auf den Haken 49 verzeichtet werden.

Zum Laden einer intraokularen Linse 13 in die Linsenfaltkartusche 11 wird wie folgt vorgegangen:

Die Linsenfaltkartusche 11 wird in die Vorrichtung 10 eingeschoben. Nach dem Einschieben der Linsenfaltkartusche 11 erstreckt sich der Haken 49 (in Fig. 1 nicht sichtbar) durch die Injektordüse 19 hindurch in den vorderen Bereich der Halbschale 21. Dann wird mit einer Hand die Ladevorrichtung 10 gehalten und der Flügel 29 nach hinten bewegt. Mit einer Pinzette oder einem anderen geeigneten Instrument wird die Linse 13, wie in Figur 1 gezeigt, in die Kartusche 11 eingelegt. Dabei wird darauf geachtet, dass die vordere Haptik 39 vom Haken 49 erfasst werden kann und die hintere Haptik 37 in der Kerbe 35 liegt. Das Beladen erfolgt bei der Herstellerfirma.

Bei der Operation entnimmt der Operateur im sterilen Bereich die Ladevorrichtung 10 zusammen mit Kartusche 11 und der noch ungefalteten Linse 13 aus der sterilen Verpackung und verschwenkt den Flügel 29 nach vom, bis er in der Schnappeinrichtung 33 einschnappt. Die Linse 13 ist nun gefaltet. Beim Herausziehen der Linsenfaltkartusche 11 (Fig. 3) wird die vordere Haptik 39 durch den Haken 49 erfasst, in die Injektordüse 19 geleitet und gleichzeitig gestreckt. Dies ist leicht überprüfbar, wenn die Linsenfaltkartusche aus durchsichtigem Kunststoff besteht.

Die Ladevorrichtung gemäss dem Ausführungsbeispiel von Fig. 4 ist grundsätzlich gleich ausgebildet wie die Ladevorrichtung gemäss den Figuren 1 und 3. Es werden daher für gleiche Teile die gleichen Bezugszeichen verwendet und es kann auch auf die vorangehende Beschreibung verwiesen werden. Die Ladevorrichtung gemäss Fig. 4 besitzt aber zusätzlich ein Verriegelungselement in Form eines um die Achse 51 verschwenkbaren Hebels 53. Am Verriegelungselement 53 ist ein Anschlag 55, der die Linsenfaltkartusche 11 in teilgeöffneter Stellung hält. Es ist dies eine Stellung, in welcher die Linse 13 am Herausfallen gehindert wird, aber sich in spannungslosem Zustand befindet. Ein weiterer Anschlag 57 hält die Linsenfaltkartusche 11 in der eingezeichneten axialen Lage. Am Verriegelungselement 53 ist ferner ein Mitnehmer 59 ausgebildet, auf dem die hintere Haptik 37 aufliegt. Wird das Verriegelungselement 53 entgegen der Kraft einer nicht dargestellten Rastvorrichtung im Uhrzeigersinn verschwenkt, fördert der Mitnehmer 59 die hintere Haptik 37 in die Kerbe 35, wie dies in Fig. 1 dargestellt ist. Die Linsenfaltkartusche 11 kann nun durch Betätigung des Flügels 29 verschlossen und, wie in Fig. 3 gezeigt, entnommen werden.

Es sind verschiedene Aenderungen der Ladevorrichtung 10 möglich, ohne vom Erfindungsgedanken abzuweichen. So kann das Verriegelungselement 53 einen Zapfen aufweisen, der in der Stellung von Fig. 4 in eine Oeffnung des Support eingreift und so ein Verschwenken des Verriegelungselementes 53 verhindert. Die Verriegelung kann dann aber gelöst werden, indem das Verriegelungselement herausgezogen wird, bis es an einen Anschlag anstösst und der Zapfen nicht mehr in Eingriff mit der Oeffnung ist.

Zusammenfassend kann folgendes festgehalten werden:

Die Vorrichtung 10 zum Beladen einer Linsenfaltkartusche 11 mit einer intraokularen Linse 13 weist einen Support 41 auf, welcher eine Aussparung 43 zur Aufnahme der noch leeren Linsenfaltkartusche 11 aufweist. Der Führung und Halterung der Linsenfaltkartusche 11 dienen eine zylindrische Oeffnung 45, welche den hinteren Teil 20 der Injektordüse 19 umfasst und eine Nut 47, welche den Flügel 27 festhält. Durch die zylindrische Oeffnung 45 erstreckt sich ein Haken 45 nach aussen. Wird somit die Linsenfaltkartusche 11 in die Vorrichtung geschoben, erstreckt sich der Haken 49 durch die Injektordüse 19 hindurch bis in den vorderen Bereich der Halbschale 21. Die Linse 13 wird nun so in die Linsenfaltkartusche 11 gelegt, dass die vordere Haptik 39 vom Haken 49 erfasst werden kann und die hintere Haptik 37 in der Kerbe 35 liegt. In dieser Form liegt dem Operateur die Vorrichtung 10 mit Linsenfaltkartusche 11 und Linse 13 steril verpackt vor. Zum Gebrauch entnimmt er die Vorrichtung 10 und verschwenkt den Flügel 29, bis er in der Schnappeinrichtung 33 einschnappt. Die Linse 13 ist nun gefaltet. Beim Herausziehen der Linsenfaltkartusche 11 wird die vordere Haptik 39 durch den Haken 49 in die Injektordüse 19 geleitet.

## Patentansprüche

1. Ladevorrichtung zum Beladen einer Linsenfaltkartusche (11) mit einer intraokularen Linse (13), **dadurch gekennzeichnet, dass** die Vorrichtung einen Support (41) aufweist, welcher eine Auflage (43) zur Aufnahme der Linsenfaltkartusche (11) besitzt.

2. Ladevorrichtung nach Anspruch 1, **dadurch gekennzeichnet, dass** der Support (41) Führungsmittel (45,47) zum Einführen der Linsenfaltkartusche (11) aufweist.

3. Ladevorrichtung nach Anspruch 2, **dadurch gekennzeichnet, dass** die Führungsmittel eine Führung, z.B. eine Oeffnung (45) aufweisen, welche die Injektordüse (19,20) führt.

4. Ladevorrichtung nach einem der Ansprüche 1 bis 3, **dadurch gekennzeichnet, dass** sie einen Haken (49) aufweist, welcher sich durch die Führung (45) für die Injektordüse (19,20) nach aussen erstreckt.

5. Ladevorrichtung nach einem der Ansprüche 2 bis 4, **dadurch gekennzeichnet, dass** die Führungsmittel eine Führung, z.B. eine Nut (47), auweisen, um das äussere Ende eines der Flügel (27) der Linsenfaltkartusche (41) zu führen.

6. Ladevorrichtung nach einem der Ansprüche 1 bis 5, **dadurch gekennzeichnet, dass** sie ein Verriegelungselement (53) aufweist, um die Linsenfaltkartusche nach dem Einlegen der intraokularen Linse (13) in teiloffener Stellung zu halten.

7. Ladevorrichtung nach Anspruch 6, **dadurch gekennzeichnet, dass** das Verriegelungselement (53) einen Mitnehmer (59) für die hintere Haptik (37) aufweist, um beim Entriegeln die hintere Haptik (37) richtig für den Injektionsvorgang zu platzieren.

8. Ladevorrichtung nach Anspruch 6 oder 7, **dadurch gekennzeichnet, dass** das Verriegelungselement (53) auf einer Achse (51) des Supports gelagert ist.

9. Ladevorrichtung nach einem der Ansprüche 6 bis 8, **dadurch gekennzeichnet, dass** das Verriegelungselement (53) als manuell betätigbarer Schwenkarm ausgebildet ist.

10. Ladevorrichtung nach einem der Ansprüche 6 bis 9, **dadurch gekennzeichnet, dass** das Verriegelungselement (53) einen Anschlag (55) aufweist, um die Linsenfaltkartusche (11) in teiloffener Stellung zu halten.

11. Linsenfaltkartusche zur Verwendung mit einer Ladevorrichtung gemäss einem der Ansprüche 1 bis 10, mit einem Ladekanal (15) bestehend aus zwei Halbschalen (21,23), an den Halbschalen (21,23) angeordneten Flügeln (27,29) und einer vom am Ladekanal (15) angeordneten Injektordüse (19), **dadurch gekennzeichnet, dass** hinten an einer der Halbschalen eine Kerbe (35) zur Aufnahme der hinteren Haptik (37) angeordnet ist.

12. Set für die Implantation einer intraokularen Linse (13), welches eine Linsenfaltkartusche (11) umfasst, **dadurch gekennzeichnet, dass** es weiter eine Ladevorrichtung (10) gemäss einem der Ansprüche 1 bis 10 zum Laden der Linsenfaltkartusche (11) umfasst.

13. Set nach Anspruch 12, **dadurch gekennzeichnet, dass** es weiter einen Injektor umfasst, in welche die Linsenfaltkartusche eingesetzt werden kann.

14. Set nach Anspruch 12 oder 13, **dadurch gekennzeichnet, dass** die Ladevorrichtung (10) mit der Linsenfaltkartusche (11) und der von der Linsenfaltkartusche (11) sicher gehaltenen, aber ungefalteten intraokularen Linse (13) in einer sterilen Verpackung gelagert sind.
